# FASCICULE DE BREVET EUROPEEN

(11) **EP 3 254 645 B1**
(45) Date de publication et mention de la délivrance du brevet: **09.01.2019**
(21) Numéro de dépôt: 17173490.8
(22) Date de dépôt: 30.05.2017
(51) Int. Cl.: A61F 2/38

(54) **PARTIE TIBIALE D'UNE PROTHÈSE DE GENOU, NOTAMMENT UNICOMPARTIMENTAIRE, COMPORTANT UN PLATEAU TIBIAL**
TIBIATEIL EINER KNIEGELENKPROTHESE, INSBESONDERE EINE EINSEITIGE KNIEGELENKPROTHESE, MIT EINER TIBIAPLATTFORM
TIBIAL PART OF A KNEE PROSTHESIS, IN PARTICULAR A UNICOMPARTMENTAL KNEE PROSTHESIS, COMPRISING A TIBIAL TRAY

(30) Priorité: 10.06.2016 FR 1670306
(43) Date de publication de la demande: 13.12.2017
(73) Titulaire: XNOV IP SARL, 1882 Luxembourg (LU)
(72) Inventeur: MARCEAUX, Pascal, 52000 Chaumont (FR)
(74) Mandataire: Eidelsberg, Olivier Nathan

(56) Documents cités:
- EP-A1- 1 693 026
- US-A- 4 795 468
- US-A1- 2005 125 068
- US-A1- 2013 218 284

## Description

La présente invention se rapporte à une prothèse de genou et plus particulièrement à un plateau tibial d'une prothèse de genou, notamment du type unicompartimentaire.

Une prothèse du genou comporte une partie tibiale et une partie fémorale. La partie fémorale est fixée au fémur, tandis que la partie tibiale est ancrée au tibia. Un ménisque ou insert tibial, en général en matière plus tendre que les parties fémorale et tibiale, est interposé entre la partie fémorale, et notamment un ou des condyles, et un plateau tibial de la partie tibiale ancrée à l'extrémité proximale du tibia.

Dans les prothèses actuelles, notamment unicompartimentaires, l'ancrage de la partie tibiale dans le tibia s'effectue classiquement d'une part par une ailette qui s'étend à partir du bord médial du plateau tibial dans le cas d'une prothèse unicompartimentaire et d'au moins un plot d'ancrage, classiquement en forme de "diabolo".

Dans les prothèses actuelles, notamment unicompartimentaires, la qualité de l'ancrage est médiocre et on aimerait en augmenter la résistance, notamment vis à vis de micro-mouvements, notamment pour les petits genoux, et en particulier chez les femmes, il arrive fréquemment que le plateau tibial se fracture ou se descelle.

On connaît de US 2005/0125068, de US 2013/0218284 ou de EP 1 693 026 une partie tibiale comportant un plateau tibial et des plots en forme de tige inclinée par rapport à la perpendiculaire au plateau. Les plots n'ont pas de tête et la nécessité de les incliner pour assurer l'ancrage rend celui-ci peu résistant à l'usage.

US 4 795 468 décrit une partie tibiale suivant le préambule de la revendication 1.

La présente invention vise à surmonter les inconvénients de l'art antérieur en mettant à disposition un plateau tibial d'une prothèse de genou, notamment unicompartimentaire, qui assure un excellent ancrage sans que la longévité de la prothèse n'en soit affectée. Suivant l'invention, une partie tibiale d'une prothèse de genou, notamment unicompartimentaire, est telle que définie à la revendication 1.

Ainsi, suivant l'invention, on parvient à positionner les plots dans une position proche du bord latéral du plateau tibial, et donc anatomiquement proches de la coupe osseuse et à proximité de l'os cortical, qui permet d'obtenir un fort effet de levier pour l'ancrage, et donc une grande stabilité de l'ancrage, sans pour autant qu'ils en viennent à fracturer l'os par manque de matière osseuse de ce dernier du côté du bord. En outre, dans le même temps on s'assure que l'ancrage tibial puisse comporter de préférence deux ou trois points d'ancrage (notamment une ailette et deux plots) qui sont répartis à la surface du plateau tibial, idéalement en étant le plus éloignés les uns des autres et de l'ailette de manière à augmenter le bras de levier entre les points d'ancrage et donc la stabilité primaire (l'interface prothèse / os étant ainsi moins sollicitée), notamment pour donner une meilleure résistance aux micro-mouvements.

Suivant un mode de réalisation préféré de l'invention, le au moins un plot est de forme tronconique en ayant une partie tronquée ou découpée sur le côté latéral tournée vers le tronçon latéral pour ainsi décaler le centre de la section d'ancrage par rapport à la section transversale à la base de la tige.

Suivant un mode de réalisation préféré de l'invention, le au moins un plot est de forme tronconique en "diabolo" tronqué ou découpé.

Suivant un mode de réalisation préféré de l'invention, la partie tibiale est une partie tibiale d'une prothèse unicompartimentaire, une ailette faisant saillie d'un tronçon médial du bord périphérique du plateau en étant plus proche du centre de la section d'ancrage que du centre de la section transversale à la base de la tige. De préférence, il est prévu deux plots, notamment tronqués.

Suivant un mode de réalisation préféré de l'invention, le au moins un plot, notamment les deux plots sont tronqués ou découpés suivant une ligne de coupe, perpendiculaire au plan du plateau, parallèle au bord latéral de la prothèse.

Suivant un mode de réalisation préféré, le au moins un plot est tronqué par la formation d'une face latérale du plot qui s'étend le long de la tige et de la tête.

De préférence, la face latérale est inclinée par rapport à la perpendiculaire (ou verticale) à la face inférieure de sorte que son bord périphérique comporte un tronçon supérieur qui est plus éloigné du tronçon latéral du bord périphérique de la face que ne l'est le point le plus bas de son bord périphérique.

En particulier, le bord supérieur de la face latérale du ou de chaque plot tronqué s'étend le long d'un arc de cercle, notamment concentrique ou parallèle avec le tronçon latéral.

La présente invention se rapporte aussi à une prothèse de genou, notamment unicompartimentaire, comportant une partie fémorale, un insert tibial et une partie tibiale suivant l'invention.

A titre d'exemple, on décrit maintenant un mode de réalisation préféré de l'invention en se reportant aux dessins, dans lesquels :
- la figure 1 est une vue de dessus d'un plateau tibial d'une prothèse unicompartimentaire suivant un mode de réalisation de l'invention ;
- la figure 2 est une vue en perspective de dessous du plateau tibial de la figure 1 ;
- les figures 3A, 3B, 3C et 3D sont des vues respectivement de dessus, de face, de côté et en perspective d'un plot de la partie tibiale des figures 1 et 2 ; et
- la figure 4 est une vue en coupe suivant la ligne A-A de la figure 3D.

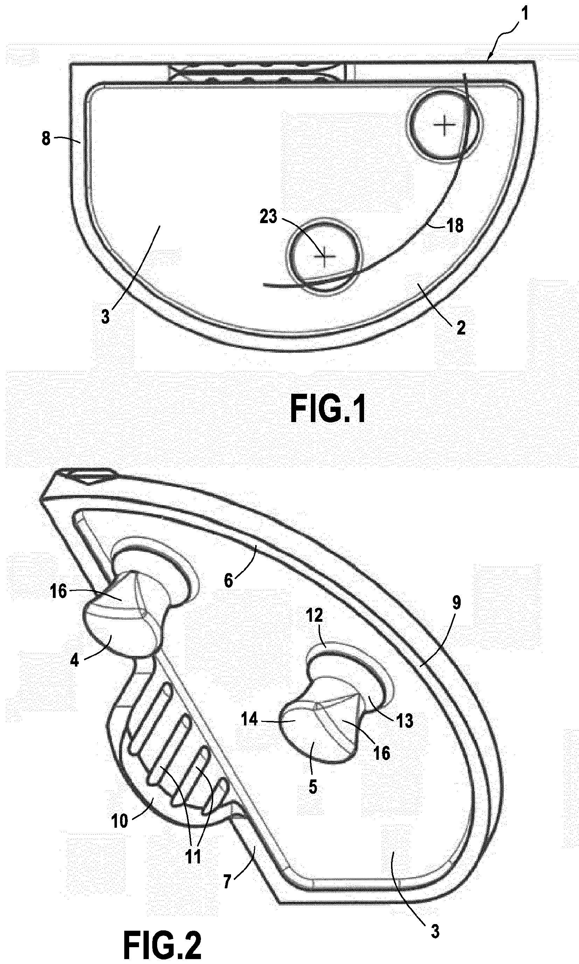

Aux figures 1 et 2, il est représenté une partie 1 tibiale d'une prothèse unicompartimentaire suivant l'invention. Les figures 1 et 2 sont des vues de dessous, c'est-à-dire vues du côté du tibia, auquel est destiné à être ancré la partie tibiale 1. La partie 1 tibiale est constitué d'un plateau 2 tibial de la face 3 inférieure duquel font saillie deux plots 4 et 5.

Une nervure 6 périphérique fait saillie du bord périphérique de la face 3 inférieure et aide à l'ancrage et au positionnement du plateau sur une face résectée proximale du tibia. La nervure 6 périphérique comporte un tronçon 7 rectiligne médial, un tronçon 8 rectiligne antérieur et un tronçon 9 incurvé, par exemple en arc de cercle, reliant les deux tronçons 7 et 8 rectilignes. Une ailette 10, comportant deux séries de rainures 11 verticales tracées respectivement du côté intérieur et extérieur, fait saillie du tronçon 7 médial, perpendiculairement au plateau, et est destinée à être ancrée dans un évidement correspondant formé dans la face résectée du tibia, les rainures recevant de la matière osseuse pour réaliser l'ancrage.

Les plots 4 et 5 ont des formes dites en plots "diabolo". Chaque plot comporte en succession un premier tronçon proximal sensiblement tronconique formant embase 13 dont la section transversale circulaire diminue vers le haut le long d'un quart de cercle jusqu'à atteindre un diamètre d1, un deuxième tronçon intermédiaire sensiblement tronconique formant tige 14 s'évasant vers le haut jusqu'à un bord 22 périphérique le plus à l'extérieur et un troisième tronçon distal formant tête 15 d'ancrage du plot qui s'étend au delà de la section transversale, dite d'ancrage, délimitée par le bord 22. L'embase 13 est de forme tronconique ou torique, ayant sa section qui diminue vers le haut.

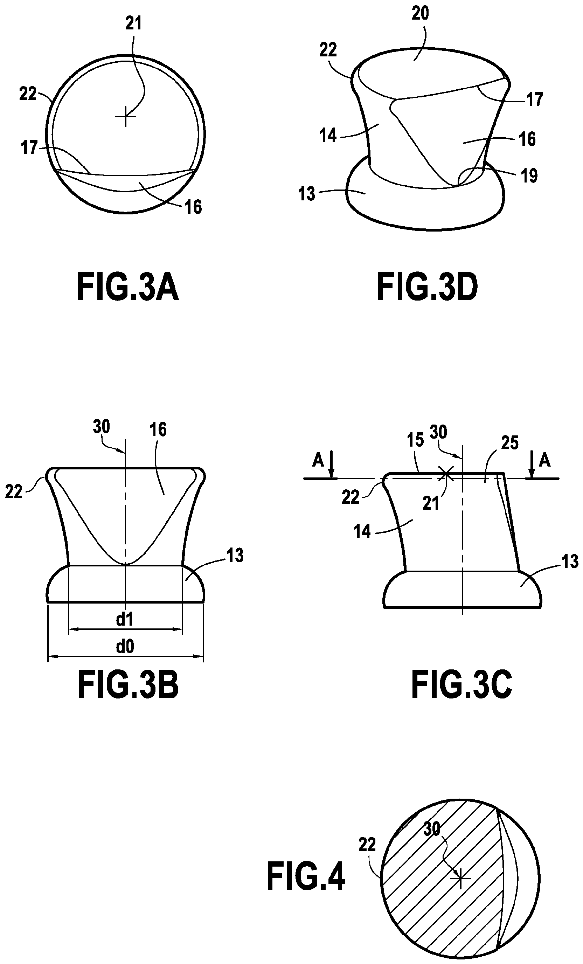

De préférence, en section longitudinale, une partie de la surface latérale de la tige 15 a une forme incurvée concave pour améliorer "l'ancrage" du plot dans le tibia. La tête 15 s'étend de la fin de la tige (c'est-à-dire à partir de la section 25 délimitée par le bord 22 le plus à l'extérieur et correspondant à la coupe suivant la ligne A-A représentée à la figure 4) jusqu'à la fin du plot, qui ici est constitué d'une surface plane 20, en ayant sa section transversale qui diminue de la section 25 jusqu'à celle de la surface plane 20.

En particulier, l'embase 13 et la tige 14 ont un axe longitudinal 30 vertical et la section 25 de délimitation ou d'ancrage entre la tige 14 et la tête 15 correspond à la section transversale perpendiculaire à l'axe 30 et passant par la ou les point (s) 22 périphérique (s) les plus à l'extérieur, c'est-à-dire les plus éloignés de l'axe 30 (la distance étant mesurée perpendiculairement à l'axe 30).

La tige 14 et la tête 15 sont tronquées. Ainsi, il est formée une face 16 latérale du plot qui s'étend entre la surface 20 plane de sommet et l'embase 13 du plot. La face 16 latérale est inclinée par rapport à la perpendiculaire (ou verticale) à la face 3 inférieure de sorte que son bord périphérique comporte un tronçon supérieur 17, qui peut notamment être rectiligne, sensiblement rectiligne, en léger arc de cercle (comme c'est le cas aux figures) ou analogue, qui est plus éloigné du tronçon 9 latéral du bord périphérique de la face 3 que ne l'est le point 19 le plus bas de son bord périphérique.

En particulier dans le mode de réalisation représenté, les bords 17 des deux plots tronqués s'étendent le long d'un même arc de cercle 18 qui est concentrique ou parallèle avec le tronçon 9.

Ainsi, le centre 23 de la section 12 circulaire (do) de la base du plot et de la section circulaire (d1) à la base de la tige 14 est plus proche du bord 9 latéral que ne l'est le centre 21 de la section transversale d'ancrage au niveau du bord 22, c'est à dire à l'interface entre la tête 15 et la tige 14.

Pour définir le centre d'une section transversale non circulaire, par exemple de la section 25 transversale d'ancrage, et de manière générale d'une surface fermée plane, on considère le centre de gravité surfacique uniforme de la surface en question. Il s'agit donc du point qui constitue le centre de gravité surfacique de la surface pour une répartition surfacique de masse uniforme.

Le centre de l'interface 25 est décalé du côté intérieur de la plaque de l'embase tibiale par rapport au centre 23 du cercle de la section 12.

## Revendications

1. Partie tibiale d'une prothèse de genou, notamment unicompartimentaire, comportant un plateau tibial ayant une face (3) inférieure, ou distale, délimitée par un bord (6) périphérique ayant au moins un tronçon (9) latéral, et de laquelle fait saillie au moins un plot (4 ; 5) d'ancrage destiné à être ancré dans le tibia, le au moins un plot (4 ; 5) comportant au moins une partie (14) formant tige ayant un axe (30) longitudinal et une partie (15) formant tête, la partie de tige et la partie de tête définissant entre elles une interface (25) qui correspond à la section transversale du plot, perpendiculairement à l'axe longitudinal, dite section d'ancrage, qui se trouve au niveau du ou des point (s) (22) de la surface latérale du plot qui est ou sont le ou les plus éloigné (s) de l'axe (30), la distance étant mesurée perpendiculairement à l'axe (30), **caractérisée en ce que** la section (d1) transversale à la base de la tige et la section (25) d'ancrage sont mutuellement décalées.

2. Partie tibiale suivant la revendication 1, **caractérisée en ce que** l'axe longitudinal est perpendiculaire à la face (3) inférieure.

3. Partie tibiale suivant la revendication 1 ou 2, **caractérisée en ce que** le centre (21) de la section (25) d'ancrage est plus éloigné du tronçon (9) latéral du plateau que le centre (23) de la section (d1) transversale à la base de la tige.

4. Partie tibiale suivant l'une des revendications 1 à 3, **caractérisée en ce que** le au moins un plot, notamment de forme cylindrique, a une partie tronquée sur le côté latéral tourné vers le tronçon (9) latéral pour ainsi décaler le centre de la section d'ancrage par rapport à la section (d1) transversale à la base de la tige.

5. Partie tibiale suivant l'une des revendications 1 à 4, **caractérisée en ce que** le au moins un plot est de forme cylindrique en "diabolo" tronqué.

6. Partie tibiale suivant l'une des revendications 1 à 5, **caractérisée en ce que** la partie tibiale est une partie tibiale d'une prothèse unicompartimentaire, une ailette (10, 11) faisant saillie d'un tronçon médial (7) du bord périphérique (6) du plateau en étant plus proche du centre (21) de la section (25) d'ancrage que du centre (23) de la section (d1) transversale à la base de la tige.

7. Partie tibiale suivant l'une des revendications 1 à 6, **caractérisée en ce que** le au moins un plot, notamment les deux plots sont tronqués suivant une ligne de coupe, perpendiculaire au plan du plateau, parallèle au bord latéral de la prothèse.

8. Partie tibiale suivant l'une des revendications 1 à 7, **caractérisée en ce que** le au moins un plot est tronqué par la formation d'une face (16) latérale du plot qui s'étend le long de la tige et de la tête.

9. Partie tibiale suivant la revendication 8, **caractérisée en ce que** la face (16) latérale est inclinée par rapport à la perpendiculaire (ou verticale) à la face (3) inférieure de sorte que son bord périphérique comporte un tronçon (17) supérieur qui est plus éloigné du tronçon (9) latéral du bord (6) périphérique de la face (3) que ne l'est le point (19) le plus bas de son bord périphérique.

10. Prothèse de genou comportant une partie fémorale, un insert tibial et une partie tibiale suivant l'une des revendications 1 à 9.

11. Prothèse de genou unicompartimentaire, comportant une partie fémorale, un insert tibial et une partie tibiale suivant l'une des revendications 1 à 9.

## Patentansprüche

1. Tibiateil einer, insbesondere unikompartimentellen, Kniegelenkprothese, welche eine Tibiaplattform mit einer unteren oder distalen Seite (3) aufweist, die von einem Umfangsrand (6) mit wenigstens einem seitlichen Abschnitt (9) begrenzt wird und von der wenigstens ein Verankerungszapfen (4; 5) vorsteht, der dazu bestimmt ist, in der Tibia verankert zu werden, wobei der wenigstens eine Zapfen (4; 5) wenigstens einen Teil (14), der einen Schaft mit einer Längsachse (30) bildet, und einen Teil (15), der einen Kopf bildet, aufweist, wobei der Schaftteil und der Kopfteil zwischen sich eine Grenzfläche (25) definieren, welche dem Querschnitt des Zapfens senkrecht zur Längsachse, Verankerungsquerschnitt genannt, entspricht, welcher sich an dem Punkt oder den Punkten (22) der Seitenfläche des Zapfens befindet, der oder die von der Achse (30) am weitesten entfernt ist oder sind, wobei der Abstand senkrecht zur Achse (30) gemessen wird, **dadurch gekennzeichnet, dass** der Querschnitt (d1) an der Basis des Zapfens und der Verankerungsquerschnitt (25) zueinander versetzt sind.

2. Tibiateil nach Anspruch 1, **dadurch gekennzeichnet, dass** die Längsachse senkrecht zu der unteren Seite (3) ist.

3. Tibiateil nach Anspruch 1 oder 2, **dadurch gekennzeichnet, dass** der Mittelpunkt (21) des Verankerungsquerschnitts (25) weiter von dem seitlichen Abschnitt (9) der Plattform entfernt ist, als der Mittelpunkt (23) des Querschnitts (d1) an der Basis des Zapfens.

4. Tibiateil nach einem der Ansprüche 1 bis 3, **dadurch gekennzeichnet, dass** der wenigstens eine Zapfen, insbesondere von zylindrischer Form, einen abgeschnittenen Teil auf der dem seitlichen Abschnitt (9) zugewandten lateralen Seite aufweist, um so den Mittelpunkt des Verankerungsquerschnitts bezüglich des Querschnitts (d1) an der Basis des Zapfens zu versetzen.

5. Tibiateil nach einem der Ansprüche 1 bis 4, **dadurch gekennzeichnet, dass** der wenigstens eine Zapfen von einer zylindrischen Form eines abgeschnittenen "Diabolos" ist.

6. Tibiateil nach einem der Ansprüche 1 bis 5, **dadurch gekennzeichnet, dass** der Tibiateil ein Tibiateil einer unikompartimentellen Prothese ist, wobei eine Rippe (10, 11) von einem medialen Abschnitt (7) des Umfangsrandes (6) der Plattform vorsteht und dabei dem Mittelpunkt (21) des Verankerungsquerschnitts (25) näher ist als dem Mittelpunkt (23) des Querschnitts (d1) an der Basis des Zapfens.

7. Tibiateil nach einem der Ansprüche 1 bis 6, **dadurch gekennzeichnet, dass** der wenigstens eine Zapfen, insbesondere die zwei Zapfen entlang einer Schnittlinie abgeschnitten sind, die senkrecht zur Ebene der Plattform und parallel zum seitlichen Rand der Prothese ist.

8. Tibiateil nach einem der Ansprüche 1 bis 7, **dadurch gekennzeichnet, dass** der wenigstens eine Zapfen durch die Ausbildung einer seitlichen Fläche (16) des Zapfens abgeschnitten ist, die sich entlang des Schaftes und des Kopfes erstreckt.

9. Tibiateil nach Anspruch 8, **dadurch gekennzeichnet, dass** die seitliche Fläche (16) bezüglich der Senkrechten (oder Vertikalen) zur unteren Seite (3) derart geneigt ist, dass ihr Umfangsrand einen oberen Abschnitt (17) aufweist, welcher von dem seitlichen Abschnitt (9) des Umfangsrandes (6) der Seite (3) weiter entfernt ist als der niedrigste Punkt (19) ihres Umfangsrandes.

10. Kniegelenkprothese, welche einen Femurteil, einen Tibiaeinsatz und einen Tibiateil nach einem der Ansprüche 1 bis 9 aufweist.

11. Unikompartimentelle Kniegelenkprothese, welche einen Femurteil, einen Tibiaeinsatz und einen Tibiateil nach einem der Ansprüche 1 bis 9 aufweist.

## Claims

1. The tibial part of a knee prosthesis, in particular unicompartimentary, comprising a tibial plate having a lower, or distal face (3), bounded by a surrounding edge (6) having at least one lateral portion (9) from which projects at least one anchoring post (4, 5) intended to be anchored in the tibia, the at least one post (4, 5) comprising at least one part (14) forming a stem having a longitudinal centre line (30) and a part (15) forming the head, the stem part and the head part together defining an interface (25) which corresponds to the transverse section of the post, perpendicular to the longitudinal centre line, called the anchoring section, which is located at the point (s) (22) on the lateral surface of the post which is, or are, furthest from the centre line (30), the distance being measured perpendicular to the centre line (30), **characterised in that** the transverse section (d1) at the base of the stem and the anchoring section (25) are mutually offset.

2. A tibial part according to claim 1, **characterised in that** the longitudinal centre line is perpendicular to the lower face (3).

3. A tibial part according to claim 1 or 2, **characterised in that** the centre (21) of the anchoring section (25) is further from the lateral portion (9) of the plate than the centre (23) of the transverse section (d1) at the base of the stem.

4. A tibial part according to one of claims 1 to 3, **characterised in that** the at least one post, in particular of a cylindrical shape, has a truncated part on the lateral side facing the lateral portion (9) thereby offsetting the centre of the anchoring section with respect to the cross section (d1) of the base of the stem.

5. A tibial part according to one of claims 1 to 4, **characterised in that** the at least one post is of cylindrical shape in a truncated "diabolo".

6. A tibial part according to one of claims 1 to 5, **characterised in that** the tibial part is a tibial part of a unicompartimentary prosthesis, a blade (10, 11) projecting from a medial portion (7) of the surrounding edge (6) of the plate being nearer to the centre (21) of the anchoring section (25) than to the centre (23) of the transverse section (d1) at the base of the stem.

7. A tibial part according to one of claims 1 to 6, **characterised in that** the at least one post, in particular the two posts, are truncated along a cutting line, perpendicular to the plane of the plate, parallel to the lateral edge of the prosthesis.

8. A tibial part according to one of claims 1 to 7, **characterised in that** the at least one post is truncated by the formation of a lateral face (16) of the post which extends along the stem and the head.

9. A tibial part according to claim 8, **characterised in that** the lateral face (16) is inclined with respect to the perpendicular (or vertical) at the lower face (3) so that its surrounding edge comprises an upper portion (17) that is further from the lateral portion (9) of the surrounding edge (6) of the face (3) which is not the lowest point (19) of its surrounding edge.

10. A knee prosthesis comprising a femoral part, a tibial insert and a tibial part according to one of claims 1 to 9.

11. A unicompartimentary knee prosthesis, comprising a femoral part, a tibial insert and a tibial part according to one of claims 1 to 9.
